# EUROPEAN PATENT APPLICATION

(11) **EP 4 289 576 A1**
(43) Date of publication of application: **13.12.2023**
(21) Application number: 22749654.4
(22) Date of filing: 31.01.2022
(51) Int. Cl.: B26B 19/48, A61B 5/00, A61B 5/053, A61B 5/0531

(54) **ELECTRIC SHAVER**

(30) Priority: 04.02.2021 JP 2021016434
(71) Applicant: Panasonic Intellectual Property Management Co., Ltd., Osaka-shi, Osaka 540-6207 (JP)
(72) Inventor: OKUDA Ichiro, Kadoma-shi Osaka 571-0057 (JP); SUGIURA Akiho, Kadoma-shi Osaka 571-0057 (JP)
(74) Representative: Grünecker Patent- und Rechtsanwälte PartG mbB
(86) International application number: PCT/JP2022/003567
(87) International publication number: WO 2022/168782

(57) **Abstract**

The present disclosure provides an electric razor capable of more accurately measuring a skin state. Electric razor (10) according to the present disclosure includes main body (20) including blade part (240) for cutting hair, and a sensor that is attached to main body (20) and measures a state of target skin (S) having hair to be cut by blade part (240).

## Description

### TECHNICAL FIELD

The present disclosure relates to an electric razor.

### BACKGROUND ART

Conventionally, as disclosed in PTL 1, an automated system for beauty that includes a data measurement group that measures data of skin (that is, one of parts to be treated for beauty), and a calculation controller that can receive the measured data and set a necessary operation condition has been proposed.

In PTL 1, various kinds of data such as moisture, temperature, and pH of the skin are measured by using measuring instruments such as a moisture measuring instrument, a temperature measuring instrument, and a pH measuring instrument. The calculation controller sets operation conditions of various beautification devices including a permanent epilator based on the measured various kinds of data (that is, the data measurement group).

By doing this, a beauty treatment including permanent hair loss can be performed under optimal conditions in accordance with a skin state.

### Citation List

### Patent Literature

PTL 1: Unexamined Japanese Patent Publication No. S60-021746

### SUMMARY OF THE INVENTION

### Technical problem

An object of the present disclosure is to provide an electric razor capable of more accurately measuring a skin state.

### Solution to problem

An electric razor according to the present disclosure includes a main body including a blade part for cutting hair, and a sensor that is attached to the main body and measures a state of a target skin having hair to be cut by the blade part.

### Advantageous effect of invention

The electric razor according to the present disclosure can measure the state of the target skin having hair to be cut by the blade part by using the sensor attached to the main body. Thus, the skin state can be more accurately measured.

### BRIEF DESCRIPTION OF THE DRAWINGS

Fig. 1 is a perspective view illustrating an electric razor according to a first exemplary embodiment.
Fig. 2 is a perspective view schematically illustrating an inner blade included in the electric razor according to the first exemplary embodiment.
Fig. 3 is a plan view schematically illustrating the electric razor according to the first exemplary embodiment.
Fig. 4 is a rear view schematically illustrating the electric razor according to the first exemplary embodiment.
Fig. 5 is a side view schematically illustrating the electric razor according to the first exemplary embodiment.
Fig. 6 is a diagram schematically illustrating an example of a method for attaching a sensor according to the first exemplary embodiment.
Fig. 7 is a plan view schematically illustrating an electrode according to a modification of the first exemplary embodiment.
Fig. 8 is a diagram for explaining an attachment position of the sensor according to the first exemplary embodiment.
Fig. 9 is a diagram schematically illustrating a use state of the electric razor according to the first exemplary embodiment.
Fig. 10 is a diagram for explaining a positional relationship between a blade surface of an outer blade, an electrode, and target skin in a use state of the electric razor according to the first exemplary embodiment.
Fig. 11 is a block diagram illustrating an example of an electric razor including a skin contact sensing unit.
Fig. 12 is a block diagram illustrating an example of an electric razor including a measured value correction unit.
Fig. 13 is a side view schematically illustrating an electric razor according to a second exemplary embodiment.
Fig. 14 is a diagram for explaining a positional relationship between a blade surface of an outer blade, an optical sensor, and target skin in a use state of the electric razor according to the second exemplary embodiment.
Fig. 15 is a side view schematically illustrating an electric razor according to a third exemplary embodiment.
Fig. 16 is a diagram for explaining a positional relationship between a blade surface of an outer blade, an image sensor, and target skin in a use state of the electric razor according to the third exemplary embodiment.
Fig. 17 is a block diagram illustrating an example of an electric razor including an image correction unit.
Fig. 18 is a block diagram illustrating an example of an electric razor including a calculation unit.
Fig. 19 is a block diagram illustrating an example of an electric razor including a sensor position controller.

### DESCRIPTION OF EMBODIMENT

### (Findings and like underlying present disclosure)

At a time when the inventors conceived of the present disclosure, when a skin state of a subject is measured, various kinds of data such as moisture, temperature, and pH of skin are measured by using a measuring instrument as disclosed in PTL 1.

In order to more accurately measure the skin state by using such a measuring instrument, it is necessary to set measurement conditions such as a pressing force of a sensor to the skin, an angle of the sensor with respect to the skin, and a distance to be constant.

However, when the skin state of the subject is measured at home or the like, it is common for the subject to use a small measuring instrument by pressing the measuring instrument against the skin with his or her hand. Thus, it has been difficult to measure the skin state in a state where the measurement condition is kept constant.

In addition, when the skin state is measured, since sensing of the skin with the sensor is hindered by hair such as body hair, there is also a problem that the skin state cannot be accurately measured. This is particularly remarkable when a skin state of a male is measured.

In addition, it is considered many men consider that it is not necessary to perform care for skin or the like until a time for measuring the skin state is separately provided even though they are interested in the care for the skin or the like.

Under such circumstances, the inventors conceived to measure the skin states by using electric razors that many men would use almost every day. As described above, when the skin state can be measured by using the electric razor, since it is possible to measure the skin state without taking time and effort and it is also possible to remove a beard or the like that hinders accurate measurement of the skin state, the skin state can be accurately measured.

With such an idea, the inventors have come to constitute the subject matter of the present disclosure.

The present disclosure provides an electric razor capable of more accurately measuring a skin state.

Hereinafter, exemplary embodiments will be described in detail with reference to the drawings. However, unnecessarily detailed description may not be described. For example, a detailed description of already well-known matters or a redundant description of substantially the same configuration may be omitted.

Note that, the accompanying drawings and the following description are only presented to help those skilled in the art fully understand the present disclosure, and are not intended to limit the subject matters described in the scope of claims.

In addition, in the following exemplary embodiments, a longitudinal direction of a grip part of the electric razor is defined as an up-down direction, a direction in which a head part protrudes from the grip part (that is, longitudinal direction of the head part) is defined as a width direction, and a direction orthogonal to the up-down direction and the width direction is defined as a front-rear direction.

In addition, in the present exemplary embodiment, a side of the grip part where the head part is arranged is defined as an upper side in the up-down direction, and a side of the grip part where a switch part is provided is defined as a front side in the front-rear direction.

### (First exemplary embodiment)

Electric razor 10 according to the present exemplary embodiment includes main body 20 having blade part 240 for cutting hair. Electric razor 10 mainly cuts a beard that is a body hair (that is, one type of hair) of a person or an animal.

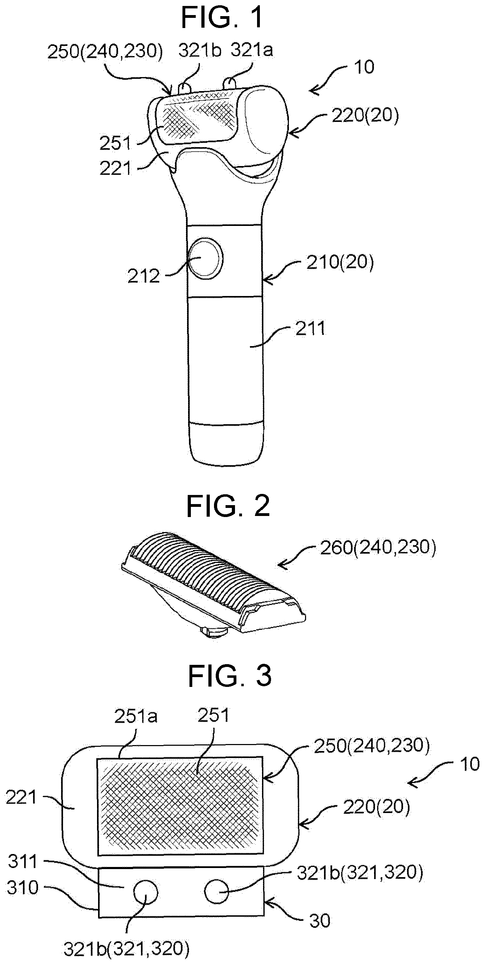

In the present exemplary embodiment, as illustrated in Figs. 1 to 5, main body 20 includes grip part 210 to be gripped by hand and head part 220 supported by grip part 210. Blade part 240 as treatment unit 230 capable of cutting hair is attached to head part 220. Preferably, blade part 240 is detachably attached to head part 220. Note that, head part 220 may not be provided in the electric razor.

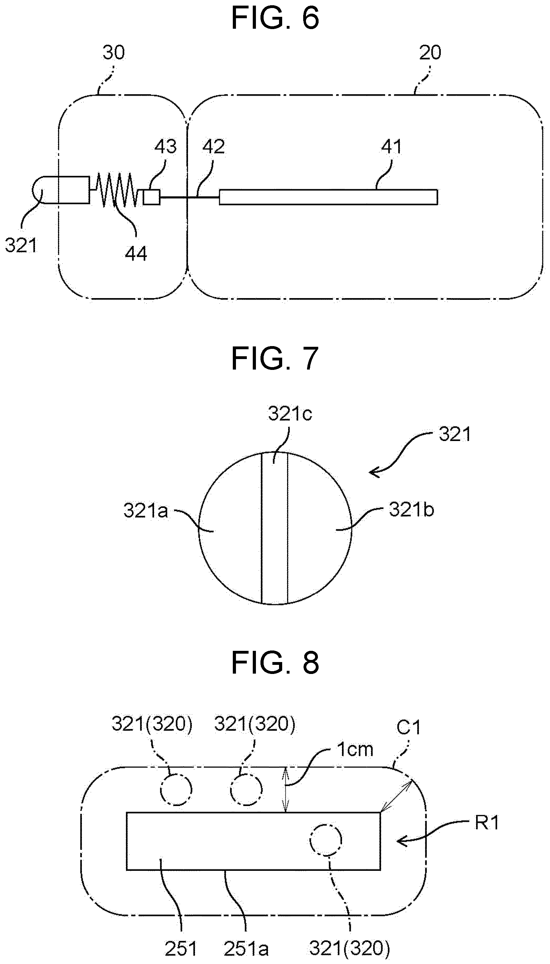

Grip part 210 is made of, for example, synthetic resin or the like, and includes grip housing 211 having a space therein, and various components such as substrate 41 (see Fig. 6) for supplying electric power for driving electric razor 10 are incorporated in grip housing 211.

In addition, push switch part 212 that operates (that is, turns on and off a power supply to) electric razor 10 is formed in grip housing 211. Note that, although push switch part 212 is illustrated as an example of the switch part in the present exemplary embodiment, a slide switch or another switch may be used as long as the switch turns on and off the power supply.

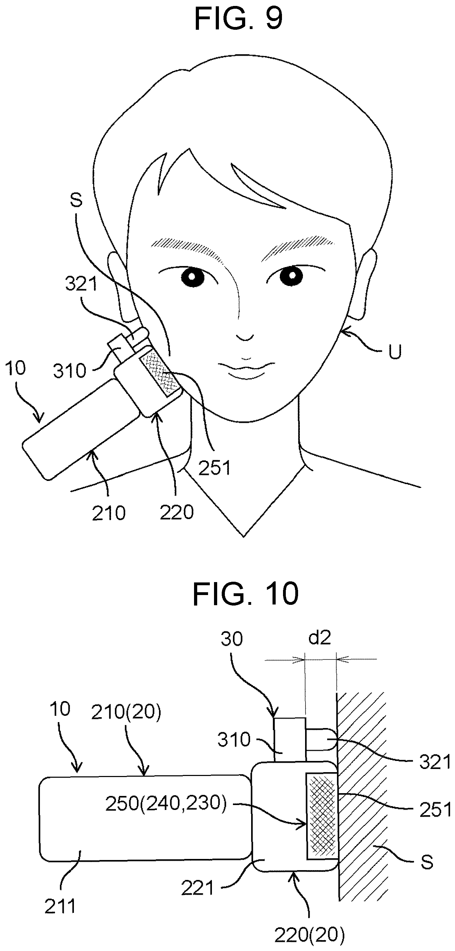

In the present exemplary embodiment, switch part 212 is formed at a front surface of grip part 210, that is, at a front (that is, forward) surface of electric razor 10. Note that, the front surface of electric razor 10 is a surface on a side where the electric razor faces user U (that is, person being treated, see Fig. 9) in a state where grip part 210 of electric razor 10 is gripped by the person being treated in normal use.

As illustrated in Fig. 1, head part 220 includes head housing 221 attached to grip part 210, and a drive mechanism (not illustrated) is accommodated in head housing 221. Conventionally and publicly known examples of the drive mechanism that can be used include an oscillatory linear actuator and a drive mechanism that includes a rotary motor and a conversion mechanism that converts a rotation motion to a linear reciprocation motion.

On the other hand, as illustrated in Figs. 1 and 2, blade part 240 includes outer blade 250 that is exposed to an upper side from head part 220, and inner blade 260 that is disposed on an inner side of outer blade 250 (that is, lower side of outer blade 250) and moves relatively to outer blade 250.

A large number of blade holes (not illustrated) are formed in outer blade 250, and outer blade 250 is arranged such that the large number of blade holes are exposed to the upper side from head part 220. A portion of outer blade 250 exposed to the upper side from head part 220 is blade surface 251 (that is, shaving surface) that comes into contact with skin (that is, target skin S) of user U (that is, person being treated). As described above, in the present exemplary embodiment, blade part 240 has blade surface 251 capable of coming into contact with target skin S when hair is cut.

On the other hand, inner blade 260 is attached to the above-described drive mechanism (not illustrated), and when the drive mechanism is driven, inner blade 260 reciprocates in the width direction (that is, in the longitudinal direction).

Such blade part 240 is provided on an upper portion of head part 220, and thus, the user can shave hair such as a beard or the like by causing skin (that is, target skin S) pressed against blade surface 251 to protrude to an inner side from the blade holes.

Here, in the present exemplary embodiment, electric razor 10 includes sensor 320, and the state of target skin S (that is, the same surface as the shaving surface) having hair to be cut by blade part 240 can be measured by using sensor 320. That is, electric razor 10 has a function as a sensor shaver including sensor 320 capable of measuring the same surface as the shaving surface.

Specifically, electric razor 10 includes sensor unit 30 that is attached to main body 20 and includes sensor 320 that measures the state of target skin S having hair to be cut by blade part 240.

Sensor unit 30 further includes sensor holding part 310 integrally molded with main body 20 to hold sensor unit 320.

In addition, in the present exemplary embodiment, sensor 320 includes electrodes 321 having positive electrode 321a and negative electrode 321b.

Electrodes 321are a so-called contact sensor that measures the state of target skin S in a state where positive electrode 321a is brought into contact with target skin S and negative electrode 321b is brought into contact with target skin S. When such electrodes 321 are used as sensor 320 that measures the state of target skin S having hair to be cut by blade part 240, for example, impedance (that is, resistance value), electric capacitance, and the like of target skin S can be measured. When the impedance (that is, resistance value), the electric capacitance, and the like of target skin S is measured, it is possible to know a moisture amount, an oil amount, and the like of target skin S.

Furthermore, in the present exemplary embodiment, electrodes 321 (that is, one element of sensor 320) are attached to main body 20 in a state where the state of target skin S can be measured when blade surface 251 (that is, blade part 240) is brought into contact with target skin S. That is, in the present exemplary embodiment, electrodes 321 (that is, one element of sensor 320) have a function as a sensor capable of measuring the state of target skin S in a use state (for example, operation posture of shaving) of electric razor 10.

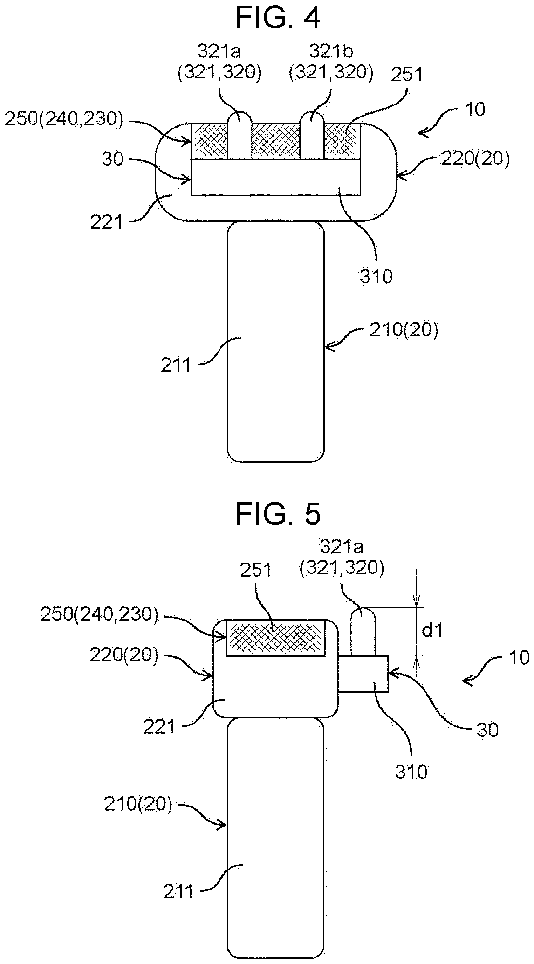

In addition, as illustrated in Fig. 6, electrodes 321 are attached to main body 20 via elastic member 44 such as a spring or rubber. Specifically, electrodes 321 are attached to main body 20 such that a distal end of each of the electrodes protrudes to the upper side from blade surface 251 in a case where the electrode is naturally held to sensor holding part 310. As illustrated in Fig. 5, at this time, a protrusion amount of each of the electrodes 321 from an upper end of sensor holding part 310 is set to predetermined protrusion amount d1.

As illustrated in Fig. 10, when blade surface 251 (that is, part of blade part 240) is brought into contact with target skin S, the protrusion amount of each of the electrodes 321 from the upper end of sensor holding part 310 is set to predetermined protrusion amount d2 which is smaller than predetermined protrusion amount dl.

As described above, in the present exemplary embodiment, each of the electrodes 321 is biased by elastic member 44 in a direction of pressing target skin S (that is, up-down direction) in a state where blade surface 251 (that is, part of blade part 240) is brought into contact with target skin S.

By doing this, electrodes 321 press target skin S with a substantially constant pressing force in a state where blade surface 251 (that is, part of blade part 240) is brought into contact with target skin S.

In addition, as illustrated in Fig. 3, inclination suppressing part 311 that suppresses an inclination of electrodes 321 from a predetermined position is provided in sensor holding part 310 (that is, one element of sensor unit 30). In the present exemplary embodiment, a state where a protrusion direction of electrodes 321 is substantially matched with the up-down direction is a predetermined position of electrodes 321. By doing this, electrodes 321 press target skin S substantially vertically in a state where blade surface 251 (that is, part of blade part 240) is brought into contact with target skin S, and thus, electrodes 321 can be more reliably brought into contact with target skin S.

As described above, in the present exemplary embodiment, inclination suppressing part 311 that suppresses the inclination of electrodes 321 from the predetermined position is provided in sensor unit 30, and thus, electrodes 321 that move while being brought into contact with target skin S have a substantially constant angle with respect to target skin S.

By doing this, a contact area of electrodes 321 (that is, one element of sensor 320) with target skin S becomes substantially constant.

Note that, two or more electrodes 321 (that is, one element of sensor 320) protruding from blade surface 251 (that is, shaving surface) themselves can have elasticity. That is, it is also possible to form two or more electrodes 321 (that is, one element of sensor 320) protruding from blade surface 251 (that is, shaving surface) with an elastic member. In addition, it is also possible to form two or more electrodes 321 (that is, one element of sensor 320) protruding from blade surface 251 (that is, shaving surface) with a spring.

In addition, in the present exemplary embodiment, as illustrated in Fig. 6, electrodes 321 are electrically connected to substrate 41 via elastic member 44, connector 43, and flexible cable 42. Electric power for driving electrodes 321 is supplied from electric power for driving electric razor 10. As described above, in the present exemplary embodiment, elastic member 44 is made of a material having conductivity.

In addition, in the present exemplary embodiment, electrodes 321 include one positive electrode 321a and one negative electrode 321b separated from positive electrode 321a. That is, one electrode of two electrodes 321 is positive electrode 321a, and the other electrode is negative electrode 321b.

Note that, as illustrated in Fig. 7 illustrating a modification of the present exemplary embodiment, a conductor may be provided on each side of insulating part 321c such that insulating part 321c is interposed therebetween, and one electrode may include positive electrode 321a and negative electrode 321b.

In addition, sensor 320 may have two or more electrodes 321 protruding from blade surface 251 (that is, shaving surface), or may have two or more sensors 320 protruding from blade surface 251 (that is, shaving surface).

In addition, as illustrated in Fig. 8, in the present exemplary embodiment, electrodes 321 (that is, one element of sensor 320) are arranged in region R1 where a position 1 cm away from outer shell 251a of blade surface 251 is outer shell C1.

By doing this, since electrodes 321 (that is, one element of sensor 320) are arranged near blade part 240 that is a basic function, the state of target skin S immediately after hair cutting (that is, immediately after the treatment by blade part 240) can be measured.

That is, blade part 240 can function as an automatic beard cutting unit that cuts an unnecessary beard so as not to cause electrodes 321 (that is, one element of sensor 320) to malfunction.

In a case where the beard of target skin S of person being treated U by using electric razor 10 having such a configuration, as illustrated in Figs. 9 and 10, blade surface 251 is slid on target skin S in a state where electrodes 321 (that is, one element of sensor 320) are brought into contact with target skin S with a substantially constant pressing force.

Thus, when electric razor 10 according to the present exemplary embodiment is used, sensor 320 can measure the state of target skin S in a state where blade part 240 cuts the beard (that is, one type of hair) of target skin S. That is, the state of target skin S (that is, the same surface as the shaving surface) can be measured while the beard of target skin S (that is, the same surface as the shaving surface) is shaved. By doing this, it is possible to measure the state of target skin S (that is, the same surface as the shaving surface) immediately after hair cutting (that is, immediately after the treatment by blade part 240).

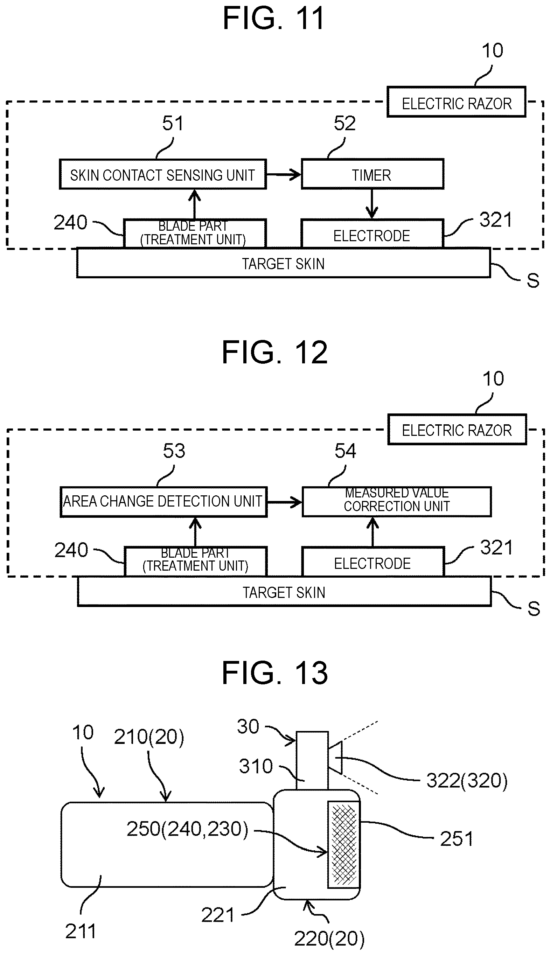

Note that, instead of measuring the state of target skin S (that is, the same surface as the shaving surface) while the beard of target skin S (that is, the same surface as the shaving surface) is shaved, the state of target skin S (the same surface as the shaving surface) may be measured before beard cutting. Hereinafter, this configuration will be described with reference to Fig. 11.

Electric razor 10 illustrated in Fig. 11 further includes skin contact sensing unit 51 that senses the contact of blade part 240 with target skin S, and timer 52 that measures a time from when skin contact sensing unit 51 senses the contact of blade part 240 with target skin S.

Sensor 320 measures the state of target skin S in a predetermined time or less after skin contact sensing unit 51 senses the contact of blade part 240 with target skin S.

The predetermined time or less can be, for example, 5 seconds or less. The predetermined time or less is more preferably 1 second or less.

As described above, in electric razor 10 illustrated in Fig. 11, the state of target skin S can be measured in a time shorter than 5 seconds after skin contact sensing unit 51 senses the contact of blade part 240 with target skin S. By doing this, the state of target skin S can be more reliably measured before hair is cut.

Note that, electric razor 10 illustrated in Fig. 11 can be configured to not only measure the state of target skin S before hair is cut, but also measure the state of target skin S even in a state where blade part 240 is cutting the beard (that is, one type of hair) of target skin S. That is, while the state of target skin S is measured before the beard is shaved, the state of target skin S can be measured while the beard is shaved.

In addition, available examples include the configuration illustrated in Fig. 12.

Electric razor 10 illustrated in Fig. 12 further includes area change detection unit 53 that detects a change in a contact area of blade part 240 with respect to target skin S, and measured value correction unit 54 that corrects a measured value measured by electrodes 321 in accordance with the change in the contact area detected by area change detection unit 53.

By doing this, the measured value measured by electrodes 321 that change in accordance with the change in the contact area of blade part 240 with target skin S is corrected by measured value correction unit 54. That is, the state of target skin S can be more accurately measured.

### (Second exemplary embodiment)

Electric razor 10 according to the present exemplary embodiment has substantially a configuration similar to the configuration electric razor 10 described in the first exemplary embodiment.

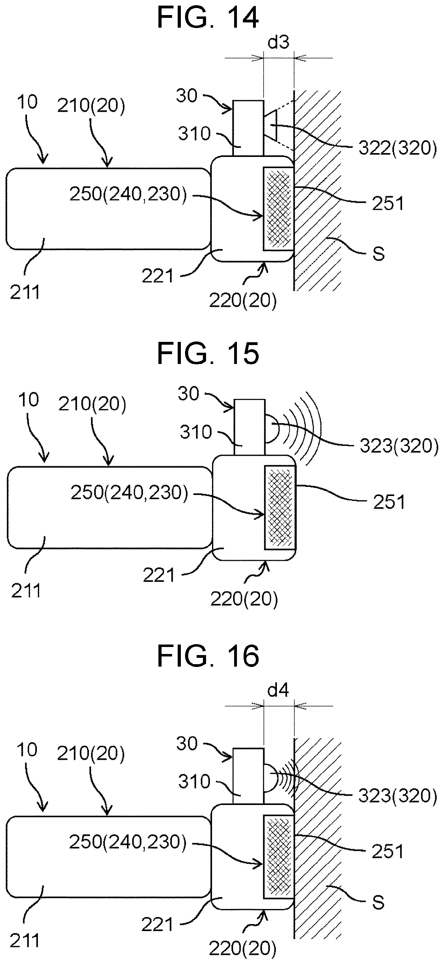

That is, as illustrated in Figs. 13 and 14, electric razor 10 according to the present exemplary embodiment includes main body 20 including blade part 240 that cuts hair, and sensor 320 that is attached to main body 20 and measures a state of target skin S having hair to be cut by blade part 240.

In addition, in the present exemplary embodiment, sensor 320 is attached to main body 20 in a state where the state of target skin S can be measured when blade part 240 is brought into contact with target skin S.

Here, in the present exemplary embodiment, sensor 320 includes optical sensor 322 that irradiates target skin S with light.

Optical sensor 322 is held (that is, fixed) to sensor holding part 310 in such a way of being separated from target skin S by predetermined distance d3 when blade part 240 is brought into contact with target skin S. That is, optical sensor 322 is held (that is, fixed) to sensor holding part 310 so as to maintain a substantially constant distance from target skin S. By doing this, more stable sensing can be performed.

Optical sensor 322 is a so-called non-contact sensor that measures the state of target skin S in a state of being separated from target skin S by predetermined distance d3. When optical sensor 322 is used as sensor 320 that measures the state of target skin S to be subjected to hair cutting by blade part 240, for example, absorptivity, reflectance, intensity change of wavelength, and the like of light applied to target skin S can be measured. Then, by measuring the absorptivity, the reflectance, the intensity change of the wavelength, and the like of the light applied toward target skin S, the hue of target skin S, the depth of the wrinkle, the deep spot, and the like can be known.

### (Third exemplary embodiment)

Electric razor 10 according to the present exemplary embodiment has substantially a configuration similar to the configuration electric razor 10 described in the first exemplary embodiment.

That is, as illustrated in Figs. 15 and 16, electric razor 10 according to the present exemplary embodiment includes main body 20 having blade part 240 that cuts hair, and sensor 320 that is attached to main body 20 and measures a state of target skin S to be subjected to hair cutting by blade part 240.

In addition, in the present exemplary embodiment, sensor 320 is attached to main body 20 in a state where the state of target skin S can be measured when blade part 240 is brought into contact with target skin S.

In the present exemplary embodiment, sensor 320 includes imaging sensor 323 that captures an image of target skin S.

Imaging sensor 323 is held (That is, fixed) to sensor holding part 310 in such a way of being separated from target skin S by predetermined distance d4 when blade part 240 is brought into contact with target skin S. That is, imaging sensor 323 is held (That is, fixed) to sensor holding part 310 so as to maintain a substantially constant distance from target skin S. By doing this, more stable sensing can be performed.

The imaging sensor 323 is a so-called non-contact sensor that measures the state of target skin S in a state of being separated from target skin S by predetermined distance d4. When such an imaging sensor 323 is used as sensor 320 that measures the state of target skin S having hair to be cut by blade part 240, for example, texture of target skin S, spot, wrinkle, and the like of a surface layer can be known from image data of target skin S.

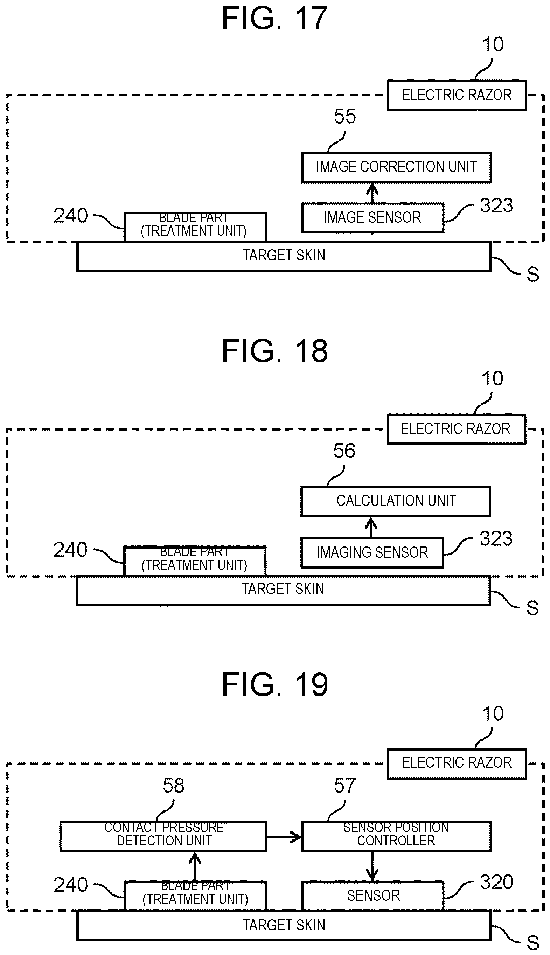

In addition, electric razor 10 may have the configuration illustrated in Fig. 17.

Electric razor 10 illustrated in Fig. 17 further includes image correction unit 55 that images a plurality of portions of target skin S by using imaging sensor 323, and corrects an image of target skin S based on pieces of imaged data of at least two portions.

By doing this, since the image of target skin S is corrected by image correction unit 55 based on the pieces of imaged data of at least two portions, more accurate image data of target skin S can be obtained.

In addition, electric razor 10 may have the configuration illustrated in Fig. 18.

Electric razor 10 illustrated in Fig. 18 further includes calculation unit 56 that images a plurality of portions of target skin S by using imaging sensor 323, and calculates three-dimensional information (hereinafter, referred to as "3D information") of target skin S based on the pieces of imaged data of at least two portions.

By doing this, since the 3D information of target skin S is calculated by calculation unit 56 based on the imaged data of at least two portions, more realistic image data of target skin S can be obtained.

In addition, electric razor 10 may have the configuration illustrated in Fig. 19.

Electric razor 10 illustrated in Fig. 19 includes sensor position controller 57 that can movably control a position of sensor 320.

Electric razor 10 illustrated in Fig. 19 further includes contact pressure detection unit 58 that detects force (that is, contact pressure) with which blade surface 251 (that is, part of blade part 240) presses target skin S, and sensor position controller 57 is configured to movably control the position of sensor 320 in accordance with the contact pressure detected by contact pressure detection unit 58.

The movable control of the position of sensor 320 can be performed by using, for example, a solenoid.

As described above, when the position of sensor 320 is movably controlled in accordance with the contact pressure detected by contact pressure detection unit 58, a distance between sensor 320 and target skin S or force for pressing target skin S in the case of the contact sensor can be set to be substantially constant when electric razor 10 is used. As a result, the skin state can be more accurately measured.

### [Actions and effects]

Hereinafter, a characteristic configuration of the electric razor described in each exemplary embodiment and the modification thereof, and an effect obtained by the characteristic configuration will be described.
(1) Electric razor 10 described in each exemplary embodiment and the modification thereof includes main body 20 including blade part 240 for cutting hair, and sensor 320 that is attached to main body 20 and measures a state of target skin S having hair to be cut by blade part 240.

When electric razor 10 is used, sensor 320 attached to main body 20 is used when the state of target skin S having hair to be cut by blade part 240. That is, electric razor 10 described in each exemplary embodiment and the modification thereof can measure the state of target skin S having hair to be cut by blade part 240 by using sensor 320 attached to main body 20. Thus, the skin state can be more accurately measured.

In addition, when electric razor 10 described in each the exemplary embodiment and the modification thereof, the user can know his or her skin state with more objective information, and can refer to information obtained by electric razor 10 when a care policy for his or her skin is determined.

(2) In addition, sensor 320 may be attached to main body 20 in a state where the state of the target skin is allowed to be measured when blade part 240 is brought into contact with target skin S.

By doing this, the state of target skin S can be measured in a state when the beard of target skin S is shaved with electric razor 10 (that is, operation posture of shaving). That is, sensor 320 can take correct measurement position and posture by simply applying electric razor 10 to target skin S as usual. As a result, it is possible to more accurately measure the skin state without feeling time and effort of measurement.

In particular, electric razor 10 is used in substantially the same time zone every day. Thus, when electric razor 10 described in (2) is used in substantially the same time zone every day, the skin state of user U (that is, person being treated) is also measured in substantially the same time zone every day. That is, the skin state can be diagnosed during a daily routine work called beard shaving. By doing this, it is possible to periodically measure the skin state in substantially the same time zone without taking time and effort.

In addition, for skin (in particular, a face part) that changes every day depending on a season, a living environment of the user, or the like, measurement needs to be periodically performed with a measuring instrument in the same time zone in order to correctly know the state thereof. However, electric razor 10 is generally used after wake-up in the morning. Thus, when electric razor 10 described in (2) is used normally, it is possible to measure target skin S in a calm state after wake-up in the morning, and to more correctly know the skin state.

Furthermore, since sensing of target skin S is performed in a state where blade surface 251 that is a relatively wide surface of electric razor 10 is brought into contact with target skin S, at least one selected from the group consisting of a distance and an angle between sensor 320 and target skin S can be set to be substantially constant. For the same reason, in the case of the contact sensor, the force pressing target skin S can be made substantially constant. As a result, the skin state can be more accurately measured.

(3) In addition, skin contact sensing unit 51 that senses contact of blade part 240 with target skin S may be further included. Sensor 320 may measure the state of target skin S in a predetermined time or less after skin contact sensing unit 51 senses the contact of blade part 240 with target skin S.

By doing this, sensor 320 can measure the state of target skin S from when skin contact sensing unit 51 senses the contact of blade part 240 with target skin S to when the cutting of the hair of target skin S is started.

(4) In addition, sensor 320 may measure the state of target skin S in a state where blade part 240 is cutting hair of target skin S.

By doing this, the state of target skin S can be measured while the beard is shaved by using electric razor 10 as usual. As a result, the state of target skin S can be measured in a state where the beard that might hinder the measurement of the state of target skin S by using sensor 320 has been removed. Thus, the state of target skin S can be more accurately measured by sensor 320.

(5) In addition, sensor 320 may include electrodes 321 including positive electrode 321a and negative electrode 321b. Electrodes 321 may measure the state of target skin S in a state where negative electrode 321b is brought into contact with target skin S while positive electrode 321a is brought into contact with target skin S.

By doing this, the impedance (that is, resistance value), the electric capacitance, and the like of target skin S can be measured, and it is possible to know a moisture amount, an oil amount, and the like of target skin S.

(6) In addition, electrodes 321 may be attached to main body 20 via elastic member 44.

By doing this, target skin S can be pressed when electrodes 321 are brought into contact with target skin S, and electrodes 321 can be more reliably brought into contact with target skin S when the state of target skin S is measured by electrodes 321.

(7) In addition, electrodes 321 may be biased by elastic member 44 in a direction of pressing target skin S in a state where blade part 240 is brought into contact with target skin S.

By doing this, electrodes 321 can be brought into contact with target skin S with substantially constant pressing force. As a result, the state of target skin S can be more accurately measured by electrodes 321.

(8) In addition, inclination suppressing part 311 that suppresses an inclination of electrodes 321 from a predetermined position may be provided.

By doing this, electrodes 321 that move while being brought into contact with target skin S can be set to have a substantially constant angle with respect to target skin S, and the state of target skin S can be more accurately measured by electrodes 321.

(9) In addition, the electric razor may further include area change detection unit 53 that detects a change in a contact area of blade part 240 with target skin S, and measured value correction unit 54 that corrects a measured value measured by electrodes 321 in accordance with the change in the contact area detected by area change detection unit 53.

In other words, the electric razor may further include electric circuitry which, in operation, detects a change in a contact area of blade part 240 with target skin S, and a corrects a measured value measured by electrodes 321 in accordance with the change in the detected contact area.

The electrical and electronic circuit includes a semiconductor element such as an integrated circuit (that is, IC) or a large scale integrated circuit (that is, LSI).

By doing this, since the measured value measured by electrodes 321 is corrected by measured value correction unit 54 in accordance with the change in the contact area detected by area change detection unit 53, the state of target skin S can more accurately be measured.

(10) In addition, sensor 320 may include optical sensor 322 that irradiates target skin S with light.

By doing this, the absorptivity, the reflectance, the intensity change of the wavelength, and the like of the light applied toward target skin S can be measured, and the hue of target skin S, the depth of the wrinkle, the deep spot, and the like can be known.

(11) In addition, when blade part 240 is brought into contact with target skin S, optical sensor 322 may be in a state of being separated from target skin S by predetermined distance d3.

By doing this, at least one selected from the distance and the angle between optical sensor 322 and target skin S can be set to be substantially constant, and the skin state can be more accurately measured.

(12) In addition, sensor 320 may include imaging sensor 323 that images an image of target skin S.

By doing this, the texture, spot, wrinkle, and the like of target skin S can be known from the image data of target skin S.

(13) In addition, when blade part 240 is brought into contact with target skin S, imaging sensor 323 may be in a state of being separated from target skin S by predetermined distance d4.

By doing this, at least one selected from the distance and the angle between imaging sensor 323 and target skin S can be set to be substantially constant, and the skin state can be more accurately measured.

(14) In addition, image correction unit 55 that corrects an image of target skin S based on pieces of imaged data of at least two portions among a plurality of portions of target skin S imaged by imaging sensor 323 may be further included.

In other words, the electric razor may further include electric circuitry which, in operation, causes imaging sensor 323 to image a plurality of portions of target skin S, and corrects an image of target skin S based on at least two pieces of imaged data.

By doing this, since the image of target skin S is corrected by image correction unit 55 based on the pieces of imaged data of at least two portions, more accurate image data of target skin S can be obtained.

(15) In addition, calculation unit 56 that calculates 3D information of target skin S based on pieces of imaged data of at least two portions among a plurality of portions of target skin S imaged by imaging sensor 323 may be further included.

In other words, the electric razor may further include electric circuitry which, in operation, causes imaging sensor 323 to image a plurality of portions of target skin S; and calculates three-dimensional information of target skin S based on pieces of imaged data of at least two portions.

By doing this, since the 3D information of target skin S is calculated by calculation unit 56 based on the imaged data of at least two portions, more realistic image data of target skin S can be obtained.

### [Others]

Although the contents of the electric razor according to the present disclosure have been described, the present disclosure is not limited to the description, and it is obvious to those skilled in the art that various modifications and improvements can be made.

For example, it is possible to appropriately combine the configurations described in each of the above exemplary embodiments and the modifications thereof to form an electric razor.

In addition, various sensors can be used as sensor 320 included in electric razor 10. For example, the electrode sensor, the non-contact sensor, the infrared sensor, the optical sensor, the image sensor, the imaging sensor, the moisture sensor, the microscope, or the like can be used as sensor 320. At this time, electric razor 10 may include two or more sensors, or may include two or more types of sensors.

In addition, the state of target skin S (hereinafter, referred to as skin information) measured by sensor 320 can be used for various purposes. For example, the measured state of target skin S (that is, skin information) can be displayed on a display such as electric razor 10 or a terminal device. In addition, it is also possible to perform control of the treatment (for example, control of adjusting the position of outer blade 250, adjusting the wavelength and intensity of light to be emitted by providing a flash lamp or the like, and the like) by treatment unit 230 based on the measured state of target skin S (that is, skin information). In addition, the measured state of target skin S (that is, skin information) can be transmitted to an external system such as a server, can be accumulated as a database, and can be used as information for proposing a more appropriate treatment.

In addition, specifications of the main body, the sensor unit, and other details (for example, a shape, a size, a layout, and the like) can be changed as appropriate.

### INDUSTRIAL APPLICABILITY

As described above, since the electric razor according to the present disclosure can more accurately measure the skin state, the electric razor can be used for various kinds of electric razors including home use and business use.

### REFERENCE MARKS IN THE DRAWINGS

- 10: electric razor
- 20: main body
- 240: blade part
- 311: inclination suppressing part
- 320: sensor
- 321: electrode
- 321a: positive electrode
- 321b: negative electrode
- 322: optical sensor
- 323: imaging sensor
- 44: elastic member
- 51: skin contact sensing unit
- 53: area change detection unit
- 54: measured value correction unit
- 55: image correction unit
- 56: calculation unit
- d3: predetermined distance
- d4: predetermined distance
- S: target skin

## Claims

1. An electric razor comprising:
a main body that comprises a blade part for cutting hair; and
a sensor that is attached to the main body and measures a state of a target skin having hair to be cut by the blade part.

2. The electric razor according to Claim 1, wherein
the sensor is attached to the main body in a state where the state of the target skin is allowed to be measured when the blade part is brought into contact with the target skin.

3. The electric razor according to Claim 2, further comprising a skin contact sensing unit that senses contact of the blade part with the target skin, wherein
the sensor measures the state of the target skin in a predetermined time or less after the skin contact sensing unit senses the contact of the blade part with the target skin.

4. The electric razor according to Claim 2 or 3, wherein
the sensor measures the state of the target skin in a state where the blade part is cutting hair of the target skin.

5. The electric razor according to any one of Claims 1 to 4, wherein
the sensor comprises electrodes including a positive electrode and a negative electrode, and
the electrodes measure the state of the target skin in a state where the negative electrode is brought into contact with the target skin while the positive electrode is brought into contact with the target skin.

6. The electric razor according to Claim 5, wherein the electrodes are attached to the main body via an elastic member.

7. The electric razor according to Claim 6, wherein the electrodes are biased in a direction of pressing the target skin by the elastic member in a state where the blade part is brought into contact with the target skin.

8. The electric razor according to Claim 6 or 7, further comprising an inclination suppressing part that suppresses an inclination of the electrode from a predetermined position.

9. The electric razor according to any one of Claims 5 to 8, further comprising:
an area change detection unit that detects a change in a contact area of the blade part with the target skin; and
a measured value correction unit that corrects a measured value measured by the electrode in accordance with the change in the contact area detected by the area change detection unit.

10. The electric razor according to any one of Claims 1 to 9, wherein the sensor comprises an optical sensor that irradiates the target skin with light.

11. The electric razor according to Claim 10, wherein the optical sensor is in a state of being separated from the target skin by a predetermined distance when the blade part is brought into contact with the target skin.

12. The electric razor according to any one of Claims 1 to 11, wherein the sensor includes an imaging sensor that images an image of the target skin.

13. The electric razor according to Claim 12, wherein the imaging sensor is in a state of being separated from the target skin by a predetermined distance when the blade part is brought into contact with the target skin.

14. The electric razor according to Claim 12 or 13, further comprising an image correction unit that corrects an image of the target skin based on pieces of imaged data of at least two portions among a plurality of portions of the target skin imaged by the imaging sensor.

15. The electric razor according to any one of Claims 12 to 14, further comprising a calculation unit that calculates three-dimensional information of the target skin based on pieces of imaged data of at least two portions among a plurality of portions of the target skin imaged by the imaging sensor.
